Europäisches Patentamt

European Patent Office

Office européen des brevets

(11) Veröffentlichungsnummer: **0 064 710**
**B2**

(12) **NEUE EUROPÄISCHE PATENTSCHRIFT**

(45) Veröffentlichungstag der neuen Patentschrift:
02.08.89

(21) Anmeldenummer: 82103746.2

(22) Anmeldetag: 03.05.82

(51) Int. Cl.⁴: **G 01 N 33/52,** C 12 Q 1/54,
**G 01 N** 31/22

(54) Mehrschichtiges Testmittel zum Nachweis einer Komponente einer flüssigen Probe.

(30) Priorität: 09.05.81 DE 3118381

(43) Veröffentlichungstag der Anmeldung:
17.11.82 Patentblatt 82/46

(45) Bekanntmachung des Hinweises auf die Patenterteilung:
11.09.85 Patentblatt 85/37

(45) Bekanntmachung des Hinweises auf die Entscheidung über den Einspruch:
02.08.89 Patentblatt 89/31

(84) Benannte Vertragsstaaten:
AT BE CH DE FR GB IT LI LU NL SE

(56) Entgegenhaltungen:
AT-B-282 832
AT-B-313 243
DE-A-2 854 342
DE-B-2 940 165
US-A-4 050 898
US-A-4 066 403

Die Akte enthält technische Angaben, die nach dem Eingang der Anmeldung eingereicht wurden und die nicht in dieser Patentschrift enthalten sind.

(73) Patentinhaber: BOEHRINGER MANNHEIM GMBH,
Patentabteilung, Abt. E Sandhofer Strasse 112-132 Postfach 31 01 20, D-6800 Mannheim 31 Waldhof (DE)

(72) Erfinder: Rothe, Anselm, Dr. rer. nat., Im Schwanklingen 20, D-6943 Birkenau (DE)
Erfinder: Selle, Adolf Karl, Lärchenweg 12, D-6943 Birkenau (DE)
Erfinder: Sojka, Bernard Karl, Dr. rer. nat., Kreuzstrasse 46, D-6804 Viernheim (DE)

## Beschreibung

Die vorliegende Erfindung betrifft Teststreifen zum Nachweis einer Komponente in einer flüssigen Probe, insbesondere in einer Körperflüssigkeit.

Teststreifen sind bereits seit langer Zeit im Gebrauch, haben jedoch in letzter Zeit insbesondere in der klinischen Chemie stark an Bedeutung gewonnen. Sie erlauben eine schnelle, einfache, halbquantitative oder quantitative Bestimmung der Komponenten einer flüssigen Probe, beispielsweise von Glucose, Eiweiß und ähnlichen in Körperflüssigkeiten wie Urin, Serum und anderen. In zunehmendem Maße werden solche Teststreifen auch von Nicht-Fachleuten verwendet, so daß es notwendig geworden ist, die Handhabung möglichst zu vereinfachen und sicherer zu gestalten.

Ein außerordentlich häufiger Fehler bei der Handhabung von solchen Teststreifen, ist das ungleichmäßige Befeuchten des Teststreifens mit der Probe durch ungenügendes Abstreifen von Probenüberschüssen nach dem Eintauchen des Teststreifens in die Probe. Auf dem Testfeld zurückbleibende Flüssigkeitstropfen führen zu einer ungleichmäßigen Verfärbung, weil entweder eine erhöhte Substanzkonzentration vorgetäuscht wird, oder durch die Flüssigkeitsreste der Zutritt von Luftsauerstoff, der als Reaktionsteilnehmer beispielsweise für alle enzymatischen Oxidationsreaktionen benötigt wird, verhindert wird, so daß zu geringe Substanzkonzentration vorgetäuscht wird. Darüber hinaus führen Flüssigkeitsreste die bei Mehrfachtesten zwischen verschiedenen Testfeldern eine Flüssigkeitsbrücke ausbilden, zu einer Übertragung von Reagenzien von einem Testfeld zum anderen, wodurch insbesondere im Randbereich der Testfelder Störreaktionen hervorgerufen werden können, die die Ablesung erschweren oder unmöglich machen. In der DE-B-2 118 455 ist deshalb vorgeschlagen worden, die aus wirtschaftlichen Gründen relativ kleinen Testbezirke, die auf einem wasserfesten Handgriff, normalerweise an einem Kunststoffstäbchen befestigt sind, mit einem hydrophobierten Papier zu unterlegen, durch welches ein Überschuß der Testflüssigkeit, der nach kurzem Eintauchen der Testvorrichtung in die Testflüssigkeit und Abstreifen der Flüssigkeit zurückbleibt, abgesaugt wird. Ein so hydrophobiertes Papier saugt über die weniger hydrophobierten Schnittkanten eventuelle Flüssigkeitsbrücken zu Nachbarbezirken relativ rasch ab und ist darüber hinaus imstande einen Flüssigkeitsüberschuß aus dem Testpapier abzusaugen, ohne jedoch die für die Reaktion notwendige Flüssigkeit zu entfernen.

In der analogen AT-B-313 243 ist darüber hinaus beschrieben, daß die Indikatorschicht auch aus einem wasserfesten Film gemäß AT-B-282 832 bestehen kann. Eine besondere, flüssigkeitsundurchlässige Schicht zwischen der Indikatorschicht und einem eventuell unterlegten Papier ist jedoch nicht vorgesehen. In der DE-A-2 854 342 sind weiterhin Teststreifen beschrieben, bei denen das Hydrophobpapier gegenüber dem Reagenzbezirk durch eine flüssigkeitsundurchlässige Schicht abgegrenzt wird, so daß lediglich Flüssigkeitsbrücken zwischen benachbarten Testbezirken aufgesaugt werden, jedoch die in und auf dem Testbezirk befindliche Flüssigkeitsmenge nicht beeinflußt wird. In den beiden vorstehenden Patenten wird als bevorzugtes Trägermaterial für das Reagenzfeld Papier verwendet, das eine relativ hohe Wasseraufnahmefähigkeit hat, so daß geringe Schwankungen der Testflüssigkeitsmenge tolerierbar sind.

In neuerer Zeit werden zunehmend als Reagenzträger dünne Polymerfilme verwendet, in die die Reagenzien eingebettet sind, da diese gegenüber Papier oder anderen faserigen Trägermaterialien eine wesentlich homogenere Struktur besitzen und somit eine gleichmäßigere Färbung im Mikrobereich und vor allem auch eine gleichmäßigere Oberfläche aufweisen, so daß die zunehmend durchgeführten photometrischen Auswertungen einer Verfärbung mit entsprechenden kleinen Remissionsphotometern durchgeführt werden können. Filme dieser Art sind beispielsweise in der DE-C-1 598 153 und der DE-B-2 332 760 beschrieben. Bei der ersten Ausführungsweise wird die Flüssigkeitsmenge dadurch reguliert, daß man einen Überschuß der Flüssigkeit aufbringt, eine definierte Zeit in den dünnen Polymerfilm eindringen läßt und einen eventuellen Überschuß gleichzeitig mit eventuellen Trübungen, die nicht in den Film eindringen, zusammen abwischt oder abwäscht. Die Beobachtung der Verfärbung kann dann von oben in gewohnter Weise erfolgen. Bei der zweiten Patentschrift wird das Problem so gelöst, daß die Flüssigkeit zunächst in eine erste hydrophile Schicht aufgesaugt wird und von dieser langsamer in einer der Saugfähigkeit der eigentlichen Reagenzschichten entsprechenden Menge an diese abgegeben wird. Die Beobachtung der Reaktion erfolgt bei dieser Vorrichtung von der Rückseite, wobei zwischen der Reagenzschicht und dem Vorratsvolumen strahlungsundurchlässige Schichten und Filterschichten dafür sorgen, daß lediglich das Reaktionsprodukt in der Reagenzschicht ausgewertet wird. Obwohl solche mehrschichtigen Vorrichtungen geringe Dosierungsschwankungen ausgleichen können, darf eine solche Vorrichtung jedoch nicht in die Testflüssigkeit eingetaucht werden, da die damit applizierte Menge "ertrinken würde". Die beiden Vorrichtungen weisen somit gegenüber den normalen, Papier verwendenden Teststreifen einen zusätzlichen "Dosierungsschritt" auf, der ihre Benutzung verkompliziert.

Es stellte sich deshalb die Aufgabe, einen Teststreifen zu entwickeln, der so einfach gehandhabt werden kann wie ein Reagenzpapiere enthaltender Streifen, jedoch die Vorteile eines Filmteststreifens aufweist, das heißt, eine homogene, nicht durch die Struktur gestörte Reaktion aufweist.

Bei Versuchen, die Vorrichtung gemäß DE-A-2 854 342 mit einem Reagenzfilm, der aus Stabilitätsgründen auf einem wasserundurchlässigen dünnen Trägerfilm befestigt ist, aufzubauen, mißlingen erwartungsgemäß, da zwar Flüssigkeitsbrücken neben dem Testbezirk in gewohnter Weise abgesaugt werden, jedoch Flüssigkeitstropfen oder -filme auf dem Testbezirk stehen bleiben und hier zu einer ungleichmäßigen Reaktion und Färbung führen. Überraschenderweise läßt sich die auf der Oberseite des Testfilms befindliche Flüssigkeitsschicht jedoch rasch und reproduzierbar durch das untergelegte Hydrophobpapier absaugen, wenn man über das Testfeld und das Hydrophobpapier ein dünnes Netz spannt, das auf beiden Seiten des Testbezirks an dem Handgriff befestigt ist. Offensichtlich wird durch Kapillarkräfte, die zwischen den Netzfäden und der Filmoberfläche wirksam werden, ein Flüssigkeitsüberschuß rasch und zuverlässig über die Kante abgeleitet und vom Hydrophobpapier aufgesaugt.

Das erfindungsgemäß mehrschichtige Testmittel zum Nachweis einer Komponente einer flüssigen Probe besteht demgemäß aus einem Träger (1), einer darauf befestigten, die flüssige Probe aufsaugenden Schicht (2), einer darüber befestigten saugfähigen Reagenzschicht (4) und einer diese überdeckende Netzschicht (5) aus einem optisch durchlässigen Material, wie Kunststoff, welche neben der aufsaugenden Schicht (2) an dem Träger (1) befestigt ist, dadurch gekennzeichnet, daß die Reagenzschicht (4) aus einer polymeren Filmschicht, in die die Reagenzien eingebettet sind, besteht und auf einem flüssigkeitsundurchlässigen Träger (3), welcher die Reagenzschicht (4) von der aufsaugenden Schicht (2) abgrenzt, befestigt ist, wobei die Saugfähigkeit der aufsaugenden Schicht (2) so bemessen ist, daß die überschüssige Probe auf dem Testbezirk innerhalb von 2 bis 10 Sekunden abgesaugt wird, wenn eine Reaktionszeit des Tests von 1 bis 2 Minuten zugrunde gelegt wird.

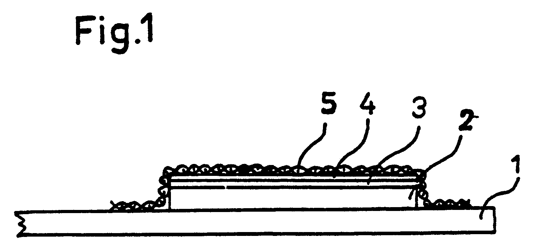

Der Aufbau der erfindungsgemäßen Testmittel ist beispielhaft in der Fig. 1 dargestellt. Die Figur zeigt das Trägerelement 1, auf dem eine saugfähige Schicht 2, einen wasserunlöslichen Filmträger 3 und den Reagenzfilm 4, wobei die beiden letzteren aus zeichnerischen Gründen wesentlich dicker gezeichnet worden sind als dies der Natur entspricht und das Abdecknetz 5.

Als Trägerfolie 1 wird normalerweise eine der im Handel befindlichen Kunststoff-Folien benutzt, jedoch kann auch ein wasserfester Karton, Metallstreifen oder ähnliches in gleicher Weise benutzt werden. Wichtig ist lediglich, daß dieser eine genügende Festigkeit besitzt, um die Handhabung zu ermöglichen, gegen die Testflüssigkeit und die verwendeten Reagenzien inert ist und eine einfache Befestigung der überdeckenden Netzschicht erlaubt.

Als aufsaugende Schicht wird vorteilhaft ein etwas hydrophobiertes Papier verwendet, jedoch können auch andere saugfähige Materialien wie offenporige Kunststoffschäume Blush-Polymere flüssigkeitsfeste Gele etc. verwendet werden. Anorganische Materialien wie beispielsweise Gips werden weniger bevorzugt, da sie normalerweise keine ausreichende Stabilität besitzen. Die Saugfähigkeit dieser Materialien sollte so bemessen sein, daß sie beim kurzzeitigen Eintauchen in die flüssige Probe (1 bis 2 Sekunden) nur äußerlich benetzt werden, jedoch Flüssigkeitsbrücken neben den Testbezirken, sowie die überschüssige Probe auf dem Testbezirk innerhalb von 2 bis 10 Sekunden absaugen, wobei eine normale Reaktionszeit des Testes von 1 bis 2 Minuten zugrunde gelegt wird. Sollte die Testzeit wesentlich länger sein, kann selbstverständlich auch die Absaugzeit, in der auf dem Testfeld noch keine idealen Bedingungen herrschen, sich entsprechend verlängern.

Als Testfilme können aus Kunststoffdispersionen hergestellte Indikatorfolien gemäß DE-C-1 598 153, "offene" Indikatorfolien gemäß DE-A-2 910 134, Blush-Polymere gemäß USA-3 555 129 oder andere, die flüssige Probe im beschränktem Maße aufnehmende, dünne, macroskopischhomogene Schichten, verwendet werden. Da solche dünnen Schichten üblicherweise nicht genügend stabil sind, so daß man sie mit den übrigen Komponenten zusammen zu dem Testmittel verarbeiten kann, empfiehlt es sich, diese Filme auf einer stabilen, dünnen, biegsamen Hilfsfolie, die normalerweise aus einem festen Kunststoff besteht, zu befestigen, oder direkt darauf zu erzeugen und beides zusammen zu verarbeiten.

Als Netzschicht können die in der DE-B-2 118 455 bereits beschriebenen Abdecknetze Verwendung finden, die üblicherweise aus Kunststoffen wie Polyamid-, Polyester- oder auch Cellulosederivaten bestehen können. Aus optischen Gründen empfiehlt es sich, relativ dünne Netze mit Fadenstärken von 20 bis 200 μm und einer offenen Lochfläche von 40 bis 80 % zu verwenden, wobei selbstverständlich das Fadenmaterial für die zur Analyse des Reaktionsproduktes benutzte Strahlung durchlässig sein muß, da die Auswertung dieser Elemente von oben her erfolgt.

Anhand der folgenden Beispiele, sollen die Vorteile der erfindungsgemäßen Testmittel näher gezeigt werden, ohne daß dadurch die Anmeldung in irgendeiner Weise beschränkt werden soll.

Die in den Beispielen angegebenen Enzymaktivitäten (U) entsprechen der int. Einheit, gemäß der ein U $10^{-6}$ Mol Substrat pro Minute bei 25°C umsetzt.

$$1 \text{ U} = 16,67 \text{ nkat} = 16,67 \times 10^{-9} \text{ mol/sec.}$$

**Beispiel 1**

Herstellung eines glucose-empfindlichen Testfilms

| 20 KU | Glucoseoxidase |
| 80 KU | Peroxidase |
| 5 ml | 1m Citratpuffer pH 5 |
| 0,13 g | Na-Alginat |
| 13 g | Polyvinylpropionat-Dispersion 50 %-ig |
| 0,375 g | Tetramethylbenzidin (3,3', 5,5') |
| 0,1 g | t-Phenylsemicarbazid |
| 1 g | Dioctylnatriumsulfosuccinat |
| 5 ml | Methoxyäthanol |
| 10 g | Kieselgel |
| 12 ml | Wasser |

werden zu einer homogenen Masse verarbeitet und mit 0,1 mm Spaltbreite auf eine 100 µm dicke Polycarbonatfolie beschichtet. Die so erhaltene Folie wird in 6 mm breite Streifen zerschnitten, die mit einem doppelseitigen Klebeband in der Nähe der einen Längskante eines 6 cm breiten Streifens aus einer etwa 0,5 mm starken PVC-Folie aufgeklebt wurde. Diese wurde danach quer zu dem aufgeklebten Streifen in 6 mm breite Stücke zerteilt.

Werden die so erhaltenen Teststreifen in glucosehaltigen Harn kurz eingetaucht, so erhält man nach einer Reaktionszeit von 1 Minute, je nach Glucosekonzentration abgestufte, grün bis blaugrüne Verfärbungen. Trotz sorgfältigem Abstreifen der Teststreifen am Rand des Probengefäßes, ist das Testfeld jedoch durch die ungleichmäßige Dicke des zurückbleibenden Flüssigkeitsfilms ungleichmäßig gefärbt, wobei durch die Verhinderung des Luftzutrittes an Stellen, auf denen sich ein dickerer Flüssigkeitsfilm befindet, die Reaktion schwächer ist. Nach einer Reaktionszeit von 5 Minuten, in der der Flüssigkeitsfilm vollständig abgetrocknet ist, zeigen sich diese Stellen aufgrund der höheren Substratkonzentration dagegen stärker verfärbt.

Eine gleichmäßige Färbung kann erreicht werden, wenn man den Teststreifen ca. 30 bis 60 Sekunden in die Probelösung eintaucht, nach dem Herausnehmen sorgfältig durch Abwischen mit weicher Watte trocknet und die Reaktion nach einer Minute abliest.

**Beispiel 2**

Herstellung der hydrophobierten Schicht

Filterpapier Schleicher & Schüll 23 SL wird mit folgender Lösung imprägniert und bei 130° getrocknet:

| 50 ml | Siliconharz HK 15a (Wacker) |
| 5 ml | Härter (10 % Aluminium-acetylaceto-nat in Toluol) |
| 1 l | Essigsäure-n-butylester |

Das so erhaltene hydrophobierte Papier wird in Analogie zu Beispiel 1 in 6 mm breite Streifen geschnitten und auf eine Polyvinylchloridfolie aufgeklebt. Mit einem weiteren Klebeband wird eine Indikatorfolie gemäß Beispiel 1 auf dem hydrophobierten Papier befestigt und wiederum quer zu diesen Bändern in 6 mm breite Streifen geschnitten.

Taucht man so erhaltene Streifen in glucosehaltigen Urin kurz ein, dann kann beobachtet werden, daß eine neben dem Testbezirk befindliche Harnbrücke rasch durch das Hydrophobpapier aufgesaugt und entfernt wird, während ein eventueller ungleichmäßiger Flüssigkeitsfilm auf dem Testfilm wie in Beispiel 1 zurückbleibt und langsam eintrocknet. Die Reaktion mit verschiedenen Glucosekonzentrationen und die ungleichmäßige Verfärbung, entsprechen vollständig den Beobachtungen gemäß Beispiel 1.

**Beispiel 3**

Herstellung eines erfindungsgemäßen Glucosetestes

Eine Polyvinylchloridfolie gemäß Beispiel 1 wird an einer Seite mit einem Schmelzkleber beschichtet, auf den ein Hydrophobpapierstreifen gemäß Beispiel 2, ein Glucosetestfilmstreifen gemäß Beispiel 1 und ein ca. 12 mm breites Nylonnetz (Fadendicke 60 µm, 45 % freie Lochfläche) übereinander gelegt werden. Mit einer erhitzten Walze, die an der Stelle des Testfilmes eine 2 mm tiefe Ausnehmung besitzt, wird das Nylonnetz auf beiden Seiten des Testfilms in den Schmelzkleber gedrückt und befestigt. Danach wird wiederum wie bei Beispiel 1 und 2 in 6 mm breite Streifen geschnitten. Testfilm und Hydrophobpapier werden in der zwischen dem Nylonnetz und der Trägerfolie gebildeten Tasche straff gehalten, können jedoch mittels einer Pinzette mit einiger Mühe herausgezogen werden.

Taucht man die so erhaltenen Teststreifen etwa eine Sekunde in Urin und streift sie leicht am Rand des Probengefäßes ab, dann kann man feststellen, daß die neben dem Testbezirk befindlichen Flüssigkeitsbrücken rasch aufgesaugt werden und die anfangs durch den zwischen den Netzmaschen befindlichen Flüssigkeitsfilm glänzende Oberfläche der Vorrichtung nach 2 bis 5 Sekunden matt wird, was darauf hindeutet, daß die Flüssigkeitsüberschüsse auch auf der Testoberfläche durch das Hydrophobpapier abgesaugt wird. Im Gegensatz zu den Testen gemäß Beispiel 1 und 2, ist die gesamte Oberfläche des Testfilmes in Abhängigkeit von der Glucosekonzentration mehr oder weniger stark, jedoch gleichmäßig gefärbt.

**Patentansprüche**

1. Mehrschichtiges Testmittel zum Nachweis einer Komponente einer flüssigen Probe, bestehend aus einem Träger (1), einer darauf befestigten, die flüssige Probe aufsaugenden Schicht (2), einer darüber befestigten saugfähigen Reagenzschicht (4) und einer diese überdeckenden Netzschicht (5) aus einem optisch durchlässigen Material, wie Kunststoff, welche neben der aufsaugenden Schicht (2) an dem Träger (1) befestigt ist, dadurch gekennzeichnet, daß die Reagenzschicht (4) aus einer polymeren Filmschicht, in die die Reagenzien eingebettet sind, besteht und auf einem flüssigkeitsundurchlässigen Träger (3), welcher die Reagenzschicht (4) von der aufsaugenden Schicht (2) abgrenzt, befestigt ist, wobei die Saugfähigkeit der aufsaugenden Schicht (2) so bemessen ist, daß die überschüssige Probe auf dem Testbezirk innerhalb von 2 bis 10 Sekunden abgesaugt wird, wenn eine Reaktionszeit des Tests von 1 bis 2 Minuten zugrunde gelegt wird.

2. Testmittel nach Anspruch 1, dadurch gekennzeichnet, daß die aufsaugende Schicht (2) aus einem hydrophobierten Papier besteht.

## Claims

1. Multi-layered test agent for the detection of a component of a liquid sample, consisting of a carrier (1), a layer (2) fixed thereupon sucking up the liquid sample, an absorbent reagent layer (4) fixed thereover and a mesh layer (5) of an optically transparent material, such as synthetic resin, covering these, which is fixed next to the absorbent layer (2) on to the carrier (1), characterised in that the reagent layer (4) consists of a polymeric film layer in which the reagents are embedded and is fixed on a liquid-impermeable carrier (3) which demarcates the reagent layer (4) from the absorbent layer (2), whereby the absorbency of the absorbing layer (2) is such that the excess sample is absorbed on the test zone within 2 to 10 seconds when the reaction time of the test is from 1 to 2 minutes.

2. A test agent according to claim 1, characterised in that the absorbent layer (2) consists of a hydrophobed paper.

## Revendications

1. Moyen d'essai pour la mise en évidence d'un composant dans un échantillon liquide, comportant un support (1), fixée sur ce support une couche (2) absorbant l'échantillon liquide, superposée et fixée sur celle-ci une couche absorbante (4) renfermant des réactifs et en recouvrement de cette dernière un filet (5) réalisé en une matière optiquement transparente telle qu'une matière synthétique, et fixé sur le support (1) à côté de la couche absorbante (2), caractérisé en ce que la couche (4) renfermant les réactifs consiste en une couche formée par un film de polymère, dans laquelle sont incorporés les réactifs, cette couche étant fixée sur un support (3) imperméable aux liquides, qui sépare la couche (4) renfermant les réactifs de la couche absorbante (2), le pouvoir d'absorption de la couche absorbante (2) étant calculé de telle façon que la fraction excédentaire de l'échantillon sur la zone d'essai soit absorbée en moins de 2 à 10 s au cas où un temps de réaction de l'essai est prévu, compris entre 1 et 2 mn.

2. Moyen d'essai selon la revendication 1, caractérisé en ce que la couche absorbante (2) est réalisée en papier rendu hydrophobe.

# Fig.1